# EUROPEAN PATENT APPLICATION

(11) **EP 2 417 919 A2**
(43) Date of publication of application: **15.02.2012**
(21) Application number: 11177141.6
(22) Date of filing: 10.08.2011
(51) Int. Cl.: A61B 17/12

(54) **Adding microscopic porosity to the surface of a microcoil to be used for medical implantation**

(30) Priority: 11.08.2010 US 854808
(71) Applicant: Micrus Endovascular Corporation, San Jose, CA 91532 (US)
(72) Inventor: Henson, Michael, Coto De Caza, California 92679 (US); Stern, Robert A., Los Altos, California 94024 (US); Watson, David A., San Jose, California 95125 (US)
(74) Representative: Small, Gary James

(57) **Abstract**

A vasoocclusive microcoil for therapeutic treatment of a patient's vasculature includes a surface with a plurality of voids or pores therein that operates to accelerate a healing process in the patient's vasculature when the microcoil is introduced into the patient's vasculature.

## Description

### CROSS-REFERENCES TO RELATED APPLICATIONS

This application is a continuation in part of Application No. 12/038,757, filed February 27, 2008, which was a continuation of Application No. 11/340,422, filed January 26, 2006, now U.S. Patent No. 7,361,367, which was based on Provisional Application No. 60/647,516, filed January 26, 2005.

### BACKGROUND OF THE INVENTION

Microcoils have been developed for implantability into aneurysms as a means of promoting healing through the obstruction of pulsatile blood flow into the center of the promoting aneurysm. Such devices have become very successful in treatment of cranial aneurysms and as a method of treating and preventing stroke when such malformations are discovered.

Another embolic coil is known which includes a distal roughened, textured surface with pockets having diameters of about 0.125 to 50 microns and depths of about 0.25 to 20 microns to provide improved platelet adhesion and to promote clotting. Another type of removable occlusion system for treating the neck of an aneurysm includes a mesh portion with pores allowing blood to flow through the mesh portion. While such embolic devices have voids or pores that can promote clotting or allow blood flow through the device, it would be desirable to provide an embolic device that can further promote healing of a patient's vasculature.

One vasoocclusive coil is known that has an enhanced therapeutic strand structure that may be formed from or incorporate therapeutic or bioactive materials such as polyglycolic acid (PGA) or poly(D,L-lactic acid-co-glycolic acid) (PGLA), or other therapeutic materials. Another embolic device is known which includes embolizing elements made of a hydrophilic, macroporous, polymeric, hydrogel foam material.

While the microcoil treatment of aneurysms is highly effective in improving the prognosis for recovery of those with such malformations, it is believed that success rate of the procedures would be enhanced if new and effective methods of treatment of the micro coils could be used to enhance the healing process once the microcoils are placed. The present invention resolves these and other limitations in prior art devices.

### SUMMARY OF THE INVENTION

The present invention treats microcoils and other implantable devices, using one or more of a variety of etching methods, which can include plasma etching, photolithography and chemical etching, to create microscopic voids in the surface of a microcoil which are complex in shape, which can act to accelerate the healing process once the coil is in place.

The present invention accordingly provides for a vasoocclusive microcoil for therapeutic treatment of a patient's vasculature, including a vasoocclusive microcoil having at least a portion having a surface defining a plurality of voids or pores therein. The present invention also provides for a method for occluding a patient's vasculature, involving the steps of providing a vasoocclusive microcoil having at least a portion having a surface defining a plurality of voids therein, and introducing the vasoocclusive microcoil into the patient's vasculature, whereby the plurality of voids or pores of the vasoocclusive microcoil can act to accelerate a healing process in the patient's vasculature. The present invention also provides for a method for forming porosity in a surface of a microcoil using one or more of a variety of etching methods, which can include plasma etching and sputtering.

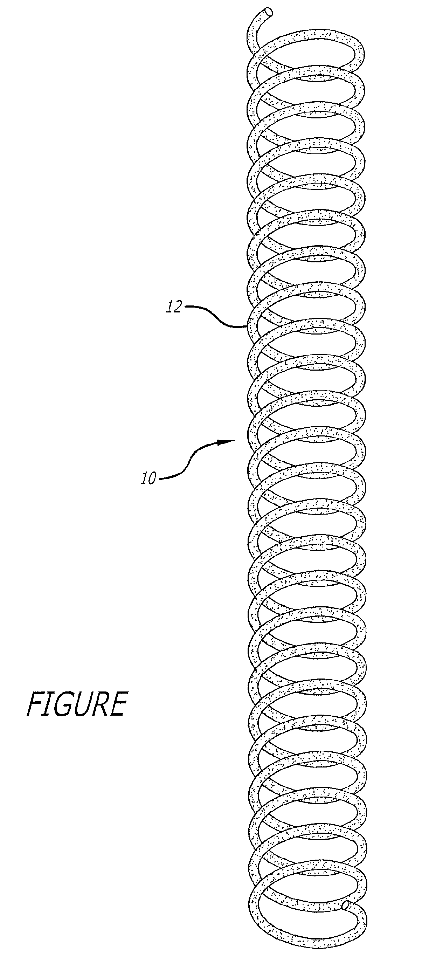

Other features and advantages of the present invention will become more apparent from the following detailed description of the preferred embodiments in conjunction with the accompanying drawing, which illustrates, by way of example, the invention.

### BRIEF DESCRIPTION OF THE DRAWING

The Figure is a perspective view of a portion of a helical single-stranded microcoil formed according to the invention.

### DETAILED DESCRIPTION OF THE PREFERRED EMBODIMENTS

As is illustrated in the drawing, which is provided for the purposes of illustration and not by way of limitation, the invention is embodied in a vasoocclusive microcoil having at least a portion with an exterior surface defining a plurality of voids or pores therein. In a presently preferred aspect illustrated in the Figure, the vasoocclusive microcoil may be formed from a single flexible strand 10 of a resilient material and/or super-elastic material, such as nickel titanium alloy, for example, and/or a radiopaque material such as platinum or gold, for example, or other similar suitable radiopaque metals. When a nickel titanium alloy is used, the nickel titanium alloy is typically heat treated such that the alloy is highly flexible at a temperature appropriate for introduction into the body via a catheter. By choosing such a material for micro-coils and the like, the devices formed from the micro-cable can be relatively easily placed into the appropriate body cavity and after placement, the device will take on a shape designed to optimize the therapeutic purposes desired for the device.

One advantageous application of the invention is for use as a vasoocclusive device formed of the vasoocclusive microcoil for insertion into aneurysms and other vascular defects for the purpose of occluding flow to the aneurysm. The single flexible strand can be formed in the shape of a helically wound coil 12, with dimensions and a configuration to fit within a micro-catheter for insertion into an area upon which a therapeutic procedure is to be performed. While helical coils are illustrated, it will be appreciated that numerous other shapes can be formed from the vasoocclusive microcoil of the invention.

As is illustrated in the Figure, the vasoocclusive microcoil includes at least a portion having an exterior surface defining a plurality of microscopic voids or pores 12 in the surface of the microcoil, which may be complex in shape. The voids or pores may be formed in the surface of the microcoil using one or more of a variety of sputtering and etching methods, which can include plasma etching, photolithography and chemical etching, for example, and any combination or combinations thereof. Other methods of creating microscopic voids or pores in the surface of an implantable device can be effective. The voids or pores can advantageously act to accelerate the healing process once the coil is in place.

It will be apparent from the foregoing that while particular forms of the invention have been illustrated and described, various modifications can be made without departing from the spirit and scope of the invention. Accordingly, it is not intended that the invention be limited, except as by the appended claims.

## Claims

1. A vasoocclusive microcoil for therapeutic treatment of a patient's vasculature, comprising:
a single flexible strand including at least a portion having an outer surface adapted to contact the tissue of a patient to be treated, the outer surface of said microcoil defining a plurality of pores having a complex shape therein that can act to accelerate the healing process once the coil is in place.

2. The vasoocclusive microcoil of Claim 1, wherein said single flexible strand is formed in the shape of a helically wound coil.

3. The vasoocclusive microcoil of Claim 1, wherein said single flexible strand comprises a super-elastic material.

4. The vasoocclusive microcoil of Claim 1, wherein said single flexible strand comprises a nickel titanium alloy.

5. The vasoocclusive microcoil of Claim 1, wherein said single flexible strand comprises a radiopaque material.

6. A method for occluding a patient's vasculature, comprising:
providing a vasoocclusive microcoil formed of a single flexible strand including at least a portion having an outer surface adapted to contact the tissue of a patient to be treated, the outer surface of said microcoil defining a plurality of pores having a complex shape therein that can act to accelerate the healing process once the coil is in place; and
introducing said vasoocclusive microcoil into the patient's vasculature.

7. The method of Claim 6, wherein said single flexible strand is formed in the shape of a helically wound coil.

8. The method of Claim 6, wherein said single flexible strand comprises a super-elastic material.

9. The method of Claim 6, wherein said single flexible strand comprises a nickel titanium alloy.

10. The method of Claim 6, wherein said single flexible strand comprises a radiopaque material.

11. The vasoclusive microcoil of claim 5 or the method of Claim 10, wherein said radiopaque material comprises platinum, or gold.

12. A method for adding microscopic porosity to the surface of a microcoil to be used for medical implantation, comprising the steps of:
providing a vasoocclusive microcoil formed of a single flexible strand including at least a portion having an outer surface adapted to contact the tissue of a patient to be treated; and
forming a plurality of pores having a complex shape in the outer surface of said microcoil that can act to accelerate the healing process once the coil is in place.

13. The method of Claim 12, wherein said step of forming a plurality of pores comprises forming said plurality of pores in the outer surface of said microcoil by plasma etching the outer surface of said microcoil.

14. The method of Claim 12, wherein said step of forming a plurality of pores comprises forming said plurality of pores in the outer surface of said microcoil by photolithography on the outer surface of said microcoil.

15. The method of Claim 12, wherein said step of forming a plurality of pores comprises forming said plurality of pores in the outer surface of said microcoil by chemical etching on the outer surface of said microcoil.
